# EUROPEAN PATENT SPECIFICATION

(11) **EP 3 084 080 B1**
(45) Date of publication and mention of the grant of the patent: **27.12.2017**
(21) Application number: 14801981.3
(22) Date of filing: 13.11.2014
(51) Int. Cl.: E02D 31/02, G01N 33/24

(54) **METHOD FOR WATERPROOFING BELOW-GRADE STRUCTURES**
VERFAHREN ZUM WASSERABDICHTEN UNTERIRDISCHER STRUKTUREN
PROCÉDÉ D'IMPERMÉABILISATION DE STRUCTURES EN DÉPRESSION

(30) Priority: 27.11.2013 IT VR20130258
(43) Date of publication of application: 26.10.2016
(73) Proprietor: THUR S.R.L., 37122 Verona (IT)
(72) Inventor: PASTOR, Mariapia, I-37021 Bosco Chiesanuova (IT); BIRTELE, Andrea, I-37138 Verona (IT)
(74) Representative: Modiano, Micaela Nadia
(86) International application number: PCT/EP2014/074454
(87) International publication number: WO 2015/078698

(56) References cited:
- WO-A1-2004/027177

## Description

The present invention relates to a method for waterproofing below-grade structures such as masonry against earth or flooring.

Techniques of (retrospective) waterproofing with injections of compounds, such as silicate mixtures, have already been used for many years, see e.g. the disclosure in WO2004/027177A1. They can be carried out both inside the masonry facing that is to be waterproofed, in order to saturate the empty spaces that are present, and at the rear of the masonry, for the purpose of creating a waterproofing barrier between masonry and soil.

The products used can be of several different types, for example: polyurethane resins, acrylic resins or silicate mixtures.

Usually, injections of resins and/or gels which are executed behind masonry for waterproofing purposes are carried out according to the following operational steps:
- drilling of the masonry according to a regular pattern;
- positioning of tubes for injection in the holes;
- carrying out of the injections starting from the areas arranged at lower vertical levels and proceeding by successive horizontal alignments toward the top of the masonry facing.

The substances injected, initially in the liquid state, solidify or gel more or less rapidly, thus becoming waterproof.

The times necessary for the injected substance to pass from the liquid state to the solid (or gel) state can vary, as a function of the type of reagent used, from a few seconds to a few hours.

The testing methods used in order to verify the success of the operation, i.e. in order to verify that the waterproofing barrier has a spatial continuity that follows the progression of the surface of the treated masonry, are generally of the indirect type and are empirical in character, and consequently they are often influenced by the practice and experience of the technician.

For the purposes of example, in the event of heavy incursions of water, one is limited to visually checking the effective interruption of the water flow through the masonry facing in order to validate the success of the operation.

If incursions of water are not present or if it is desired to provide a continuous waterproof barrier in order to block, for example, future rises in the aquifer, one is limited to using some contrivances during the step of injecting the product.

According to a fairly consolidated practice, an injection sequence is carried out that involves proceeding, for vertical masonry, by successive rows, starting at the bottom and proceeding upwardly, and stopping the insertion of the material when the injected product flows out of the lateral holes, and in particular from those positioned at greater heights.

Even with the use of such contrivances in the installation of the product, it cannot be ruled out that some areas of masonry will not be reached by the injected product, which may arrange itself only at the areas of greatest permeability.

Such problems are present to a greater extent for treatments carried out below horizontal surfaces.

The aim of the present invention is to solve the above mentioned problems and overcome the drawbacks, by providing a method for waterproofing below-grade structures, such as masonry against earth or flooring, which makes it possible to effectively waterproof a below-grade structure while verifying, at the same time, the effective continuity of the waterproofing barrier.

Within this aim, an object of the invention is to make available a method for waterproofing below-grade structures such as masonry against earth or flooring which makes it possible to intervene in order to supplement the operation in a targeted manner even at a later stage if it is found that some areas are missing the waterproofing barrier.

This aim and these and other objects which will become better apparent hereinafter are achieved by a method for waterproofing below-grade structures such as masonry against earth or flooring according to claim 1.

Further characteristics and advantages of the invention will become better apparent from the description of some preferred, but not exclusive, embodiments of the method for waterproofing below-grade structures such as masonry against earth or flooring according to the invention, which are illustrated by way of non-limiting example in the accompanying drawings wherein:

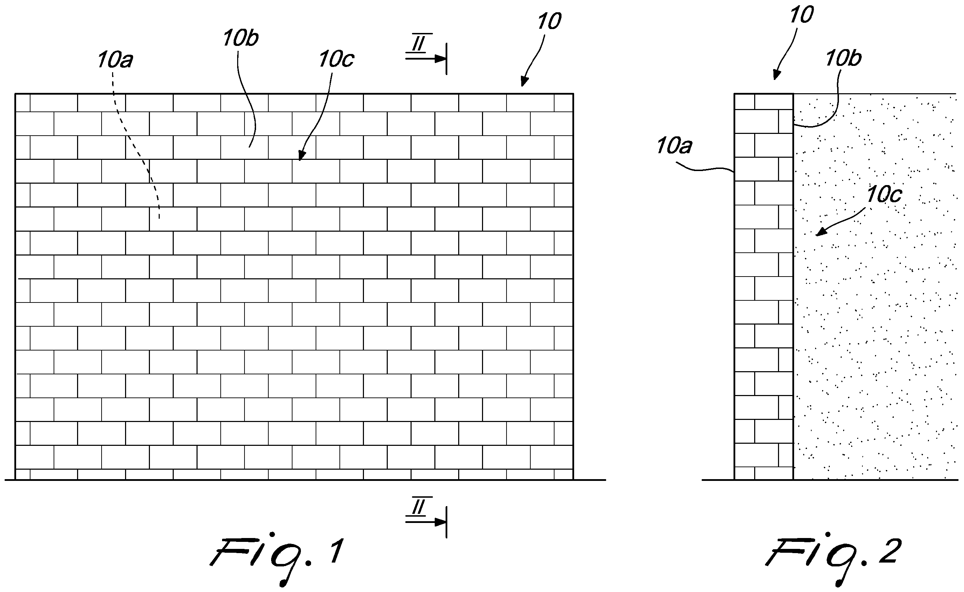

Figure 1 is a view from the outside of a below-grade structure to be waterproofed;
Figure 2 is a cross-sectional view of the masonry structure taken along the line II-II in Figure 1;

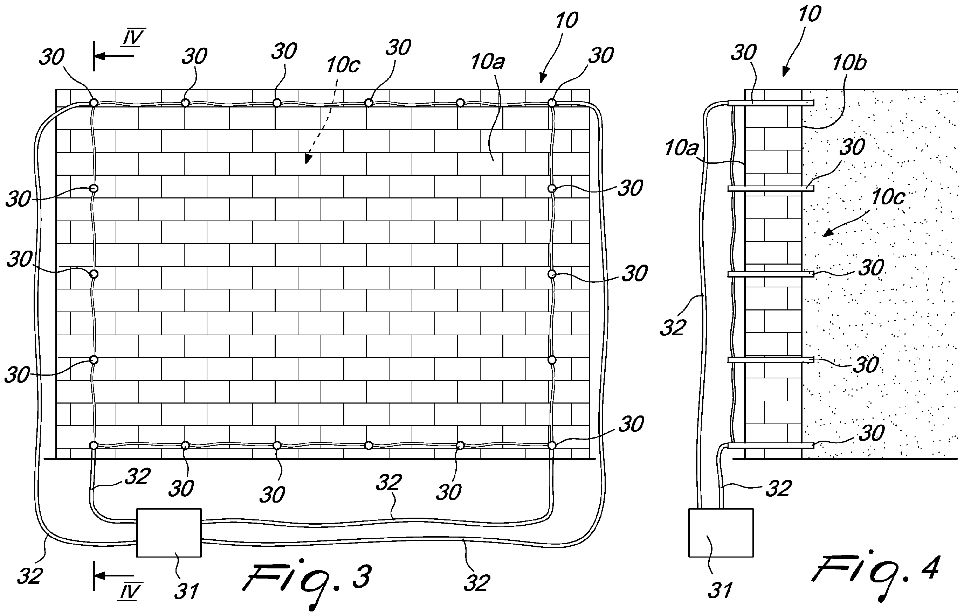

Figure 3 is a view from the inside of the masonry in Figure 1 into which electrodes have been inserted, through the masonry, for detecting the resistivity of the earth adjacent to of the masonry;
Figure 4 is a cross-sectional view of the below-grade structure taken along the line IV-IV in Figure 3;

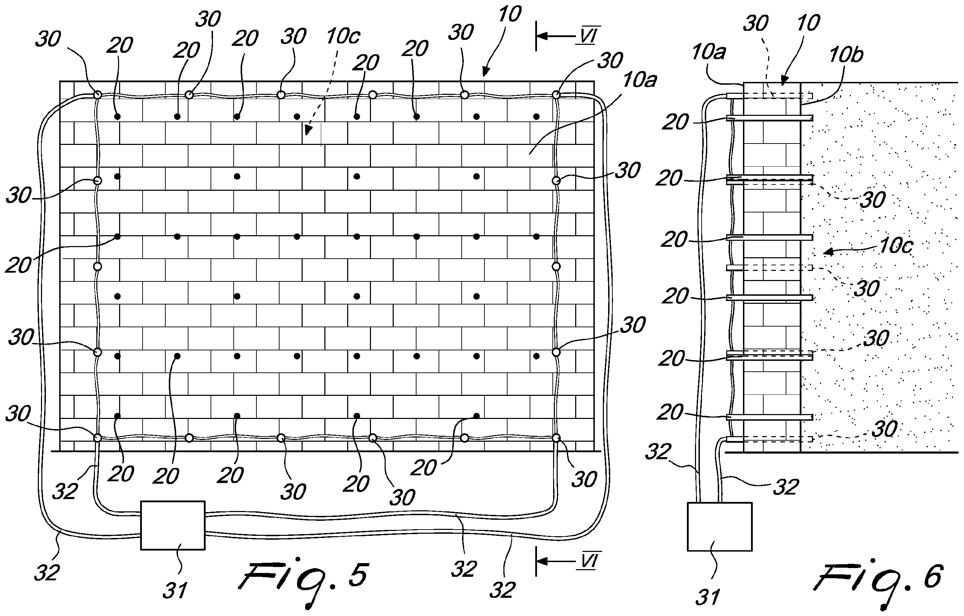

Figure 5 is a view similar to that of Figure 3 in which first injection tubes have been inserted;
Figure 6 is a cross-sectional view of the below-grade structure taken along the line VI-VI in Figure 5;

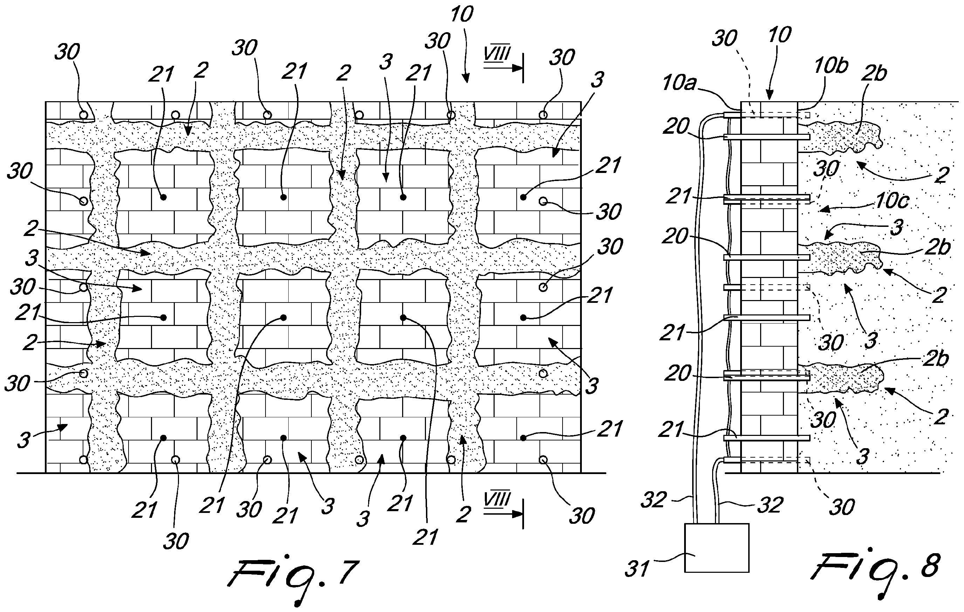

Figure 7 is a view similar to that of Figure 1 in which a first compound has been injected through the first injection tubes;
Figure 8 is a cross-sectional view of the below-grade structure taken along the line VIII-VIII in Figure 7;

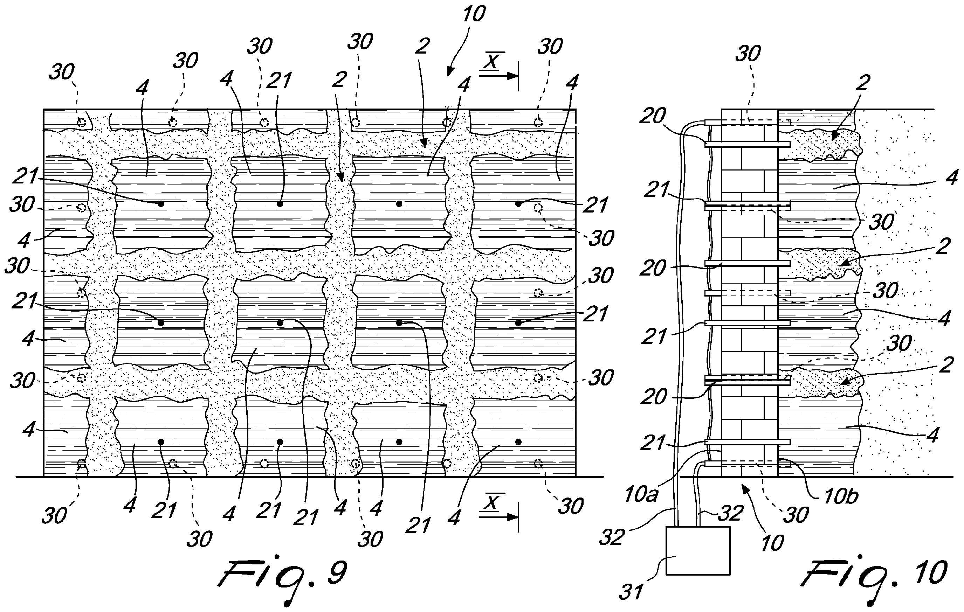

Figure 9 is a view similar to that of Figure 7 in which the second injection tubes have been inserted through the below-grade structure, and in which a second compound has been injected through the second tubes.
Figure 10 is a cross-sectional view of the below-grade structure taken along the line X-X in Figure 9.

In the embodiments illustrated, individual characteristics shown in relation to specific examples may in reality be interchanged with other, different characteristics, existing in other embodiments.

With reference to the figures, the present invention relates to un method for waterproofing below-grade structures 10 such as, for example, masonry against earth or flooring.

The method comprises:
- at least one step of injecting at least one compound 2, 4 adjacent to the surface 10b that is directed toward the outside of a region of soil 10c arranged adjacent to a below-grade structure 10 to be waterproofed;
- at least one step of measuring the electrical resistivity of the region of soil 10c subjected to the injection step.

In particular, the measurement step is adapted to detect the portions of the region of soil which are affected by the injection of the compound or compounds 2, 4.

Advantageously, the compound and/or the compounds 2, 4 injected in order to obtain the waterproofing of the below-grade structure 10 has a higher resistivity than water.

Preferably, such compound(s) 2, 4 has/have a resistivity that is at least ten times greater than the resistivity of water.

According to a preferred embodiment, the method involves a first step of measuring the electrical resistivity of the region of soil to be waterproofed before performing the injection step and at least a second step of measuring the electrical resistivity of the same region of soil 10c after carrying out the injection step.

Specifically, the method comprises: a step of comparison between the values of the electrical resistivity of respective portions of the region of soil 10c to be waterproofed, which are measured during the first measurement step and during the second measurement step in order to detect whether each portion has been affected by the injection of compound(s) 2, 4.

With reference to the embodiment shown in the figures, the compound 2, 4 comprises an expanding compound 2 and/or a diffusing compound 4.

The expanding compound 2 can be selected from the group comprising:
- a polyurethane resin;
- a urea resin;
- a silicone foam;
or mixtures thereof, while the diffusing compound 4 can be selected from the group comprising:
- a silicate mixture;
- a polyester resin;
- an epoxy resin;
or mixtures thereof.

It is possible for the injection step to be executed at first portions of the region of soil to be waterproofed which have a lower resistivity than a first reference value.

Such first portions can, for example, be identified as a consequence of the measurement step and, specifically, of the first measurement step.

Especially if diffusing or permeating compounds are used, it is advisable that these compounds have components and/or additives that are adapted to increase the resistivity of the compound.

Such components and/or additives can be adapted to increase the resistivity of the compound when such compund solidifies or gels.

For the purposes of example, such components and/or additives can be selected from the group comprising:
- acrylates;
- acrylic copolymers;
or mixtures thereof.

Conveniently, the step of measuring the electrical resistivity of the region of soil that has been subjected to the injection step comprises a step of insertion, through respective openings for passing through the masonry and along the perimeter of the region of soil to be waterproofed, of a plurality of detection electrodes 30 that are connected, for example by way of connecting cables 32, to a georesistivity meter 31.

Advantageously, the detection electrodes 30 have a covering made of insulating material at least at the portion that is designed, during use, to arrange itself along the transverse extension of the masonry so as to prevent reading errors owing to points of contact between the outer surface of the detection electrode 30 and the masonry.

In particular, the georesistivity meter 31 comprises a device for administering current into the ground connected, for example, to a battery or to a current generator.

The georesistivity meter 31 is likewise connected to the plurality of detection electrodes 30 and, in particular, to at least two current electrodes and to at least two power electrodes.

The georesistivity meter further has an apparatus for measuring the intensity of the current input into the ground by way of the current electrodes and of the potential difference between the power electrodes.

Advantageously, the step of measuring the electrical resistivity of the region of soil that has been subjected to the injection step uses a plurality of detection electrodes 30, for example sixteen, which are distributed along a profile at a close distance (generally a few dozen centimeters).

By way of an adapted device, based on one or more multi-electrode cables and corresponding switching or exchange devices, the detection electrodes 30 are connected to the data acquisition unit/energizer of the georesistivity meter 31 so as to be able to function alternatively as current electrodes or as potential electrodes.

In this manner the measurements along a profile can proceed automatically according to the desired sequence, thus providing the resistivity values, including at different depths and locations along such profile.

Inter alia, since the object of the method is to measure the resistivity of the portion of ground immediately adjacent to the masonry, it has been found that it is possible to obtain, while limiting the measuring of the resistivity to a thickness of earth of a few dozen centimeters, extremely precise values that make it possible to identify, if present, the portions of earth that are not affected by the injections.

With reference to the figures, the method can involve:
- a step of injecting an expanding compound 2 adjacent to the surface 10b directed toward the outside of a masonry structure 10 to be waterproofed so as to define at least one portion of confinement;
- a step of injecting a diffusing, or permeating compound 4, which is designed to solidify or gel adjacent to the surface 10b directed toward the outside of the masonry structure 10 and at a completion region 3 that is delimited by the confinement portions.

Conveniently, the method can involve, in sequence, the step of injecting the expanding compound 2 and the step of injecting the diffusing or permeating compound 4.

Delving deeper into the details, the method comprises: a step of insertion of first injection tubes 20 of the expanding compound 2 and a step of insertion of second injection tubes 21 of the diffusing compound 4.

The first injection tubes 20 and the second injection tubes 21 can be inserted from inside the building, by drilling the below-grade structure 10 from the surface directed toward the inside 10a, in order to emerge, with the dispensing tip thereof, adjacent to the surface 10b directed toward the outside of the below-grade structure 10 to be waterproofed.

In this case, the first injection tubes 20 and the second injection tubes 21 extend along a longitudinal direction that is substantially perpendicular to the plane of arrangement of the below-grade structure 10 to be waterproofed.

Alternatively, the first injection tubes 20 and the second injection tubes 21 can be inserted from the outside of the building in order to emerge adjacent to the surface 10b directed toward the outside of the below-grade structure 10 to be waterproofed.

In this case, the first and second injection tubes extend along a longitudinal direction that is substantially parallel to the plane of arrangement of the below-grade structure 10 to be waterproofed.

Once the injection of the compound or compounds has been completed, the method involves the removal of the first and second injection tubes 20, 21.

The first tubes 20 can likewise be left in place because their use is often rendered impossible by the fact that the expanding compound also solidifies inside them.

The method according to the invention can involve, subsequently to the injection step and to the measurement step, a further injection step at the portions where the resistivity measured is lower than the first reference value or than a second reference value.

In this case, in fact, it is probable that during the preceding injection step or steps, the compound has not reached such portions.

In practice it has been found that in all the embodiments the invention has achieved the intended aim and objects.

In practice the materials employed may be any, according to requirements.

Moreover, all the details may be substituted by other, technically equivalent elements.

The disclosures in Italian Patent Application No. VR2013A000258 from which this application claims priority are incorporated herein by reference.

Where the technical features mentioned in any claim are followed by reference numerals and/or signs, those reference numerals and/or signs have been included for the sole purpose of increasing the intelligibility of the claims and accordingly, such reference numerals and/or signs do not have any limiting effect on the interpretation of each element identified by way of example by such reference numerals and/or signs.

## Claims

1. A method for waterproofing below-grade structures (10), which comprises:
- at least one step of injecting at least one compound (2, 4) adjacent to the surface (10b) that is directed toward the outside of a region of soil (10c) arranged adjacent to a below-grade structure (10) to be waterproofed;
- at least one step of measuring the electrical resistivity of said at least one region of soil (10c) subjected to said injection step, said at least one measurement step being adapted to detect the portions of said region of soil (10c) that are affected by the injection of said at least one compound (2, 4).

2. The method for waterproofing below-grade structures (10) according to claim 1, **characterized in that** said compound (2, 4) has a higher resistivity than water.

3. The method for waterproofing below-grade structures (10) according to one or more of the preceding claims, **characterized in that** said at least one step of measuring the electrical resistivity comprises a first step of measuring the electrical resistivity of said at least one region of soil (10c) before said at least one injection step and at least one second step of measuring said electrical resistivity of said region of soil (10c) after said at least one injection step, said method comprising a step of comparison between the values of the electrical resistivity of respective portions of said region of soil (10c) which are measured during said first measurement step and during said second measurement step in order to detect whether each one of said portions has been affected by the injection of said compound (2, 4).

4. The method for waterproofing below-grade structures (10) according to one or more of the preceding claims, **characterized in that** said compound (2, 4) comprises an expanding compound (2) and/or a diffusing compound (4).

5. The method according to one or more of the preceding claims, **characterized in that** said expanding compound (2) is selected from the group comprising:
- a polyurethane resin;
- a urea resin;
- a silicone foam;
or mixtures thereof.

6. The method according to one or more of the preceding claims, **characterized in that** said diffusing compound (4) is selected from the group comprising:
- a silicate mixture;
- a polyester resin;
- an epoxy resin;
or mixtures thereof.

7. The method according to one or more of the preceding claims, **characterized in that** said injection step is performed at portions of said region of soil (10c) that have a lower resistivity than a first reference value.

8. The method according to one or more of the preceding claims, **characterized in that** said step of measuring the electrical resistivity of said at least one region of soil (10c) subjected to said injection step comprises a step of insertion, through respective openings for passing through a masonry and along the perimeter of said region of soil (10c) to be waterproofed, of a plurality of detection electrodes (30) connected to a georesistivity meter (31).

9. The method according to one or more of the preceding claims, **characterized in that** said detection electrodes (30) have a covering made of insulating material at least at the portion that is intended, during use, to arrange itself along the transverse extension of said masonry.

10. The method according to one or more of the preceding claims, **characterized in that** said at least one compound (2, 4) comprises components and/or additives adapted to increase the resistivity of the respective compound (2, 4).

11. The method according to one or more of the preceding claims, **characterized in that** said components and/or additives are adapted to increase the resistivity of the respective compound (2, 4) when it solidifies or gels.

12. The method according to one or more of the preceding claims, **characterized in that** said compounds and/or additives are selected from the group comprising:
- acrylates;
- acrylic copolymers;
or mixtures thereof.

## Patentansprüche

1. Ein Verfahren zum Wasserdichtmachen unterirdischer Strukturen (10), das Folgendes umfasst:
- mindestens einen Schritt des Einspritzens mindestens einer Verbindung (2, 4) angrenzend an die Oberfläche (10b), die der Außenseite eines Bereichs der Erde (10c) zugewandt ist, der sich angrenzend an eine wasserdicht zu machende unterirdische Struktur (10) befindet;
- mindestens einen Schritt des Messens des spezifischen elektrischen Widerstandes des mindestens einen Bereichs der Erde (10c), der dem Einspritzschritt unterzogen wird, wobei der mindestens eine Messschritt geeignet ist, die Abschnitte des Bereichs der Erde (10c) zu erfassen, die vom Einspritzen der mindestens einen Verbindung (2, 4) betroffen sind.

2. Das Verfahren zum Wasserdichtmachen unterirdischer Strukturen (10) gemäß Anspruch 1, **dadurch gekennzeichnet, dass** die Verbindung (2, 4) einen größeren spezifischen Widerstand hat als Wasser.

3. Das Verfahren zum Wasserdichtmachen unterirdischer Strukturen (10) gemäß einem oder mehreren der obigen Ansprüche, **dadurch gekennzeichnet, dass** der mindestens eine Schritt des Messens des spezifischen elektrischen Widerstandes einen ersten Schritt des Messens des spezifischen elektrischen Widerstandes des mindestens einen Bereichs der Erde (10c) vor dem mindestens einen Einspritzschritt und mindestens einen zweiten Schritt des Messens des spezifischen elektrischen Widerstandes des Bereichs der Erde (10c) nach dem mindestens einen Einspritzschritt umfasst, wobei das Verfahren einen Schritt des Vergleichs zwischen den Werten des spezifischen elektrischen Widerstands entsprechender Abschnitte des Bereichs der Erde (10c), die während des ersten Messschritts und während des zweiten Messschritts gemessen werden, zur Erfassung, ob jeder der Abschnitte vom Einspritzen der Verbindung (2, 4) betroffen ist, umfasst.

4. Das Verfahren zum Wasserdichtmachen unterirdischer Strukturen (10) gemäß einem oder mehreren der obigen Ansprüche, **dadurch gekennzeichnet, dass** die Verbindung (2, 4) eine expandierende Verbindung (2) und/oder eine diffundierende Verbindung (4) umfasst.

5. Das Verfahren gemäß einem oder mehreren der obigen Ansprüche, **dadurch gekennzeichnet, dass** die expandierende Verbindung (2) aus der Gruppe gewählt ist, die Folgendes umfasst:
- ein Polyurethanharz;
- ein Harnstoffharz;
- einen Silikonschaum
oder Mischungen davon.

6. Das Verfahren gemäß einem oder mehreren der obigen Ansprüche, **dadurch gekennzeichnet, dass** die diffundierende Verbindung (4) gewählt ist aus der Gruppe, die Folgendes umfasst:
- eine Silikatmischung;
- ein Polyesterharz;
- ein Epoxidharz
oder Mischungen davon.

7. Das Verfahren gemäß einem oder mehreren der obigen Ansprüche, **dadurch gekennzeichnet, dass** der Einspritzschritt in Abschnitten des Bereichs der Erde (10c) durchgeführt wird, die einen niedrigeren spezifischen Widerstand haben als ein erster Referenzwert.

8. Das Verfahren gemäß einem oder mehreren der obigen Ansprüche, **dadurch gekennzeichnet, dass** der Schritt des Messens des spezifischen elektrischen Widerstandes des mindestens einen Bereichs der Erde (10c), der dem Einspritzschritt unterzogen wird, einen Schritt des Einführens, durch entsprechende Öffnungen zum Durchdringen eines Mauerwerks und entlang dem Umfang des Bereichs der Erde (10c), der wasserdicht gemacht werden soll, einer Vielzahl von Erfassungselektroden (30) umfasst, die an ein Georesistivity-Messgerät angeschlossen sind.

9. Das Verfahren gemäß einem oder mehreren der obigen Ansprüche, **dadurch gekennzeichnet, dass** die Erfassungselektroden (30) zumindest an dem Abschnitt, der dazu bestimmt ist, im Gebrauch entlang der Querausdehnung des Mauerwerks angeordnet zu werden, eine Abdeckung aus Isoliermaterial haben.

10. Das Verfahren gemäß einem oder mehreren der obigen Ansprüche, **dadurch gekennzeichnet, dass** die mindestens eine Verbindung (2, 4) Komponenten und/oder Zusatzstoffe umfasst, die geeignet sind, den spezifischen Widerstand der jeweiligen Verbindung (2, 4) zu erhöhen.

11. Das Verfahren gemäß einem oder mehreren der obigen Ansprüche, **dadurch gekennzeichnet, dass** die Komponenten und/oder Zusatzstoffe geeignet sind, den spezifischen Widerstand der jeweiligen Verbindung (2, 4) zu erhöhen, wenn sie erstarrt oder geliert.

12. Das Verfahren gemäß einem oder mehreren der obigen Ansprüche, **dadurch gekennzeichnet, dass** die Verbindungen und/oder Zusatzstoffe aus der Gruppe gewählt sind, die Folgendes umfasst:
- Acrylate;
- Acryl-Copolymere
oder Mischungen davon.

## Revendications

1. Procédé d'imperméabilisation de structures en dépression (10), qui comprend :
- au moins une étape d'injection d'au moins un composé (2, 4) de manière adjacente à la surface (10b) qui est dirigée vers l'extérieur d'une région de sol (10c) agencée de manière adjacente à une structure en dépression (10) à imperméabiliser ;
- au moins une étape de mesure de la résistivité électrique de ladite au moins une région de sol (10c) soumise à ladite étape d'injection, ladite au moins une étape de mesure étant apte à détecter les parties de ladite région de sol (10c) qui sont affectées par l'injection dudit au moins un composé (2, 4).

2. Procédé d'imperméabilisation de structures en dépression (10) selon la revendication 1, **caractérisé en ce que** ledit composé (2, 4) a une résistivité supérieure à celle de l'eau.

3. Procédé d'imperméabilisation de structures en dépression (10) selon une ou plusieurs des revendications précédentes, **caractérisé en ce que** ladite au moins une étape de mesure de la résistivité électrique comprend une première étape de mesure de la résistivité électrique de ladite au moins une région de sol (10c) avant ladite au moins une étape d'injection et au moins une deuxième étape de mesure de ladite résistivité électrique de ladite région de sol (10c) après ladite au moins une étape d'injection, ledit procédé comprenant une étape de comparaison entre les valeurs de la résistivité électrique de parties respectives de ladite région de sol (10c) qui sont mesurées pendant ladite première étape de mesure et pendant ladite deuxième étape de mesure afin de détecter si chacune desdites parties a été affectée par l'injection dudit composé (2, 4).

4. Procédé d'imperméabilisation de structures en dépression (10) selon une ou plusieurs des revendications précédentes, **caractérisé en ce que** ledit composé (2, 4) comprend un composé expansible (2) et/ou un composé diffusant (4).

5. Procédé selon une ou plusieurs des revendications précédentes, **caractérisé en ce que** ledit composé expansible (2) est choisi dans le groupe comprenant :
- une résine de polyuréthane ;
- une résine d'urée ;
- une mousse de silicone ;
ou leurs mélanges.

6. Procédé selon une ou plusieurs des revendications précédentes, **caractérisé en ce que** ledit composé diffusant (4) est choisi dans le groupe comprenant :
- un mélange de silicates ;
- une résine de polyester ;
- une résine époxy ;
ou leurs mélanges.

7. Procédé selon une ou plusieurs des revendications précédentes, **caractérisé en ce que** ladite étape d'injection est effectuée dans des parties de ladite région de sol (10c) qui ont une résistivité inférieure à une première valeur de référence.

8. Procédé selon une ou plusieurs des revendications précédentes, **caractérisé en ce que** ladite étape de mesure de la résistivité électrique de ladite au moins une région de sol (10c) soumise à ladite étape d'injection comprend une étape d'introduction, par des ouvertures respectives permettant de passer à travers une maçonnerie et le long du périmètre de ladite région de sol (10c) à imperméabiliser, d'une pluralité d'électrodes de détection (30) reliées à un dispositif de mesure de la géorésistivité (31).

9. Procédé selon une ou plusieurs des revendications précédentes, **caractérisé en ce que** lesdites électrodes de détection (30) présentent un revêtement constitué d'une matière isolante au moins dans la partie qui est destinée, en service, à s'agencer le long de l'extension transversale de ladite maçonnerie.

10. Procédé selon une ou plusieurs des revendications précédentes, **caractérisé en ce que** l'au moins un composé (2, 4) comprend des composants et/ou des additifs aptes à augmenter la résistivité du composé respectif (2, 4).

11. Procédé selon une ou plusieurs des revendications précédentes, **caractérisé en ce que** lesdits composants et/ou des additifs sont aptes à augmenter la résistivité du composé respectif (2, 4) lorsqu'il se solidifie ou se gélifie.

12. Procédé selon une ou plusieurs des revendications précédentes, **caractérisé en ce que** lesdits composants et/ou des additifs sont choisis dans le groupe comprenant :
- les acrylates ;
- les copolymères acryliques ;
ou leurs mélanges.
